(19) Europäisches Patentamt
European Patent Office
Office européen des brevets

(11) EP 3 261 727 B1

(12) **FASCICULE DE BREVET EUROPEEN**

(45) Date de publication et mention de la délivrance du brevet:
**03.07.2019 Bulletin 2019/27**

(21) Numéro de dépôt: **16713518.5**

(22) Date de dépôt: **19.02.2016**

(51) Int Cl.:
***A61K 8/9706*** *(2017.01)* ***A61Q 19/08*** *(2006.01)*

(86) Numéro de dépôt international:
**PCT/FR2016/050389**

(87) Numéro de publication internationale:
**WO 2016/135400 (01.09.2016 Gazette 2016/35)**

(54) **OBTENTION D'UN EXTRAIT DE GAMÉTOPHYTES ISSUS DE L'ALGUE BRUNE ET SON UTILISATION EN TANT QUE PRINCIPE ACTIF COSMÉTIQUE ANTI-ÂGE**

GEWINNUNG EINES EXTRAKTS AUS GAMETOPHYTEN AUS BRAUNEN ALGEN UND VERWENDUNG DES BESAGTEN EXTRAKTS ALS KOSMETISCHER ANTI-AGING-WIRKSTOFF

OBTAINING AN EXTRACT FROM BROWN ALGAE GAMETOPHYTES, AND USE OF SAID EXTRACT AS A COSMETIC ANTI-AGING ACTIVE PRINCIPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.02.2015 FR 1551545**

(43) Date de publication de la demande:
**03.01.2018 Bulletin 2018/01**

(73) Titulaires:
- **Société d'Exploitation de Produits pour les Industries Chimiques SEPPIC**
  **75321 Paris cedex 07 (FR)**
- **BiotechMarine**
  **22260 Quemper Guezennec (FR)**

(72) Inventeurs:
- **CATTUZZATO, Laetitia**
  **81100 Castres (FR)**
- **DUMONT, Sandy**
  **81200 Caucalieres (FR)**
- **LE GELEBART, Erwan**
  **22620 Ploubazlanec (FR)**
- **LOEUIL, Jérôme**
  **75018 Paris (FR)**

(56) Documents cités:
**WO-A1-2015/071477 FR-A1- 2 948 877**

- **RORRER G L ET AL: "Production of hydroxy fatty acids by cell suspension cultures of the marine brown alga Laminaria saccharina", PHYTOCHEMISTRY, PERGAMON PRESS, GB, vol. 46, no. 5, 1 novembre 1997 (1997-11-01), pages 871-877, XP004293495, ISSN: 0031-9422, DOI: 10.1016/S0031-9422(97)81273-3**
- **VALERY M. DEMBITSKY: "Betaine ether-linked glycerolipids: Chemistry and biology", PROGRESS IN LIPID RESEARCH, vol. 35, no. 1, 1 mars 1996 (1996-03-01), pages 1-51, XP055174465, ISSN: 0163-7827, DOI: 10.1016/0163-7827(95)00009-7**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet un nouvel extrait de gamétophytes d'algue brune, le procédé pour sa préparation ainsi que son utilisation comme agent antivieillissement de la peau du corps humain, et les compositions cosmétiques, pharmaceutiques, dermo-pharmaceutiques à usage topique en comprenant, destinées à prévenir le vieillissement la peau du corps humain.

La peau humaine constituant la première image offerte au regard d'autrui, l'amélioration de son aspect est souvent un sujet de préoccupation pour l'être humain. La peau est le reflet soit d'un état de bien-être, souvent associé à la jeunesse, soit d'un état de fatigue et/ou de vieillissement. Les consommateurs de produits cosmétiques recherchent donc des solutions pour atténuer et/ou pour prévenir les manifestations visibles liées dudit vieillissement.

Ce vieillissement cutané est observé au niveau des différents tissus cutanés et se caractérise par des altérations métaboliques, fonctionnelles, cellulaires, architecturales et tissulaires, conduisant à des effets externes visibles comme l'apparition et l'accroissement de rides, un teint terne et/ou un manque d'uniformité de ce teint (dyschromie) ou encore la modification de la texture et des propriétés biomécaniques de la peau.

Ce vieillissement cutané résulte d'une part de facteurs propres à chaque individu (caractéristiques du patrimoine génétique propre à chaque individu) et d'autre part de facteurs environnementaux. Parmi les facteurs environnementaux qui peuvent provoquer le vieillissement cutané, il y a l'exposition répétée et prolongée aux rayonnements ultra-violets naturels ou artificiels (ou photo-vieillissement), à la pollution atmosphérique, à la fumée de cigarette, divers stress oxydants, psychologiques, émotionnels et/ou nerveux.

Le photo-vieillissement provoque des altérations de la peau à différents niveaux notamment l'élastose solaire, qui se caractérise par des modifications profondes dans l'architecture et l'organisation des fibres élastiques du derme, induisant la formation de rides très profondes et marquées, un aspect de peau tannée, à savoir raide, craquelée et brunie, ainsi que des modifications des propriétés mécaniques de ces fibres.

Face à ces agressions extérieures, la peau possède ses propres systèmes de défense, et notamment des systèmes de réparation des dommages provoqués à l'ADN par lesdites agressions extérieures. Plus particulièrement, il s'agit de systèmes antioxydants et de systèmes de dégradation des protéines non fonctionnelles. Parmi les systèmes antioxydants, deux types d'éléments antioxydants sont présents dans l'organisme: des éléments non enzymatiques, tels que la vitamine E et la vitamine C, et des éléments enzymatiques, tels que la superoxyde dismutase et la catalase. Il a notamment été démontré que certains éléments extérieurs tels que les rayonnements ultraviolets de type A et les stress thermiques chroniques, régulaient l'activité de la catalase en diminuant cette dernière et que celle-ci était reliée à l'apparition de rides [Shin et al., "Chronic heat treatment causes skin wrinkle formation and oxidative damage in hairless mice", 2012, Mech Ageing Dev, 133(2-3):92-8], [Corstjens et al., « Prevention of oxidative damage that contributes to the loss of bioenergetic capacity in ageing skin », 2007, Exp Gerontol, 42(9):924-9].

Le vieillissement de la peau a également été décrit comme étant lié à un manque de réactivité de l'organisme face au phénomène d'hypoxie (diminution du taux d'oxygène). La réponse cellulaire à l'hypoxie passe par la surexpression du facteur de transcription HIF-1, composé de 2 sous-unités: Alpha, sous-unité inductible et Beta, sous-unité exprimée de façon constitutionnelle. Ce facteur de transcription permet l'expression d'autres gènes impliqués dans l'adaptation à l'hypoxie conduisant : à un changement métabolique de la voie aérobie à la voie anaérobie, permettant de préserver la quantité d'oxygène tissulaire, à l'angiogenèse, à la survie cellulaire et dans certains cas précis à l'induction de tumeurs. Ainsi, HIF-1 voit son expression diminuer au cours du vieillissement et un état pseudo-hypoxique s'installer au sein des cellules. La Sirtuine-1 (SIRT-1) est une enzyme avec une fonction déacétylase qui a été décrite comme contrôlant l'activité du facteur de transcription HIF-1a. De part son activité, SIRT-1 est impliqué dans la régulation de nombreux processus biologiques en modifiant le niveau d'acétylation des histones et/ou des facteurs de transcription. Ainsi, son implication dans la protection anti-oxydante (via l'induction de la catalase SOD) et dans la survie cellulaire et la longévité sont largement décrits.

Il en résulte qu'une surexpression du facteur de transcription HIF-1 et/ou de la Sirtuine-1 constituent des moyens de prévenir et/ou de traiter le vieillissement de le peau du corps humain, et plus particulièrement de prévenir et/ou de traiter les effets visibles dudit vieillissement par exemple les rides, le teint terne, le manque d'uniformité du teint (dyschromie), la rigidité de la peau du corps humain, causé par le vieillissement naturel ou par une exposition prolongée au soleil, et plus particulièrement à une exposition aux rayonnements ultra-violets, ou par une exposition à des stresses oxydants.

La classe des algues brunes, encore appelée phéophycées, fait partie de l'embranchement des Ochrophytes. Cet embranchement rassemble les algues dont les cellules contiennent des pigments caroténoïdes « surnuméraires », comme la fucoxanthine, en plus des pigments chlorophylliens : chlorophylles a et c.

La classe des algues brunes comprend les ordres des : *Ascoseirales, Asterocladales, Desmarestiales, Dictyotales, Dictyotophycidae, Discosporangiales, Discosporangiophycidae, Ectocarpales, Fucales, Fucophycidae, Ishigeales, Ishigeophycidae, Laminariales, Nemodermatales, Onslowiales, Phaeophyceae ordo incertae sedis, Phaeosiphoniellales, Ralfsiales, Scytothamnales, Sphacelariales, Sporochnales, Syringodermatales, Tilopteridales.*

Tous les organismes photosynthétiques utilisent des pigments pour capter l'énergie de la lumière, habituellement une forme de la chlorophylle. La chlorophylle standard est la chlorophylle a et elle est essentielle pour le transfert de l'énergie capturée de la lumière aux molécules qui vont utiliser cette énergie. La plupart des organismes chlorophylliens ont d'autres pigments pour capter plus de lumière mais l'énergie doit toujours être transférée à la molécule de chlorophylle a.

Les algues brunes utilisent plusieurs types de pigments surnuméraires comme la chlorophylle c et les caroténoïdes. Les phéophycées présentent de grandes quantités de caroténoïdes dans leurs plastes et ce sont ces pigments bruns et jaunes qui leur donnent cette couleur brune caractéristique. Le pigment caroténoïde le plus important chez les algues brunes est la fucoxanthine qui absorbe les longueurs d'ondes de 450 à 580nm.

Les pigments caroténoïdes possèdent une structure aliphatique ou alicyclique. Ils sont liposolubles, ce qui favorise leur intégration directe dans certaines membranes. De ce fait leur solubilité dans l'eau ne peut se produire que lorsqu'ils sont liés à d'autres molécules. Les caroténoïdes sont également appelés pigments accessoires puisqu'ils doivent transférer l'énergie qu'ils capturent à la chlorophylle a. Ces pigments sont connus du grand public par le carotène qui a donné son nom à cette famille de pigments.

La fucoxanthine, qui appartient à la classe des caroténoïdes non-provitamin A est représentée par la formule :

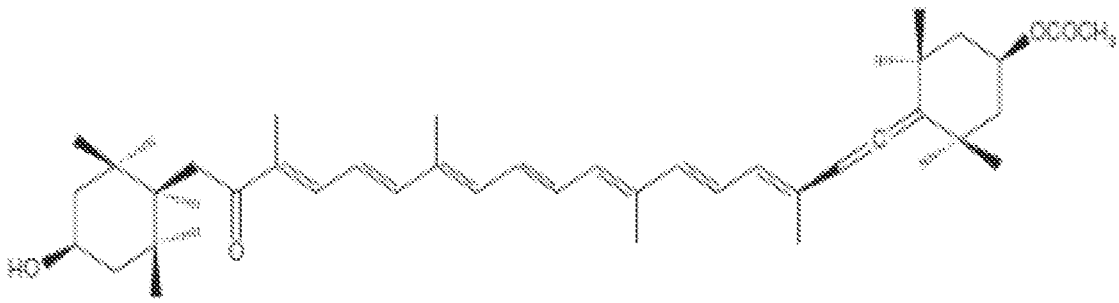

Les algues les plus décrites pour leur contenu en fucoxanthine sont *Sargassum horneri, Hizikia fusiformis, Laminaria japonica* et *Undaria pinnatifida* [Kim et Pangestuti, « Biological activities and potential health benefits of fucoxanthin derived from marine brown algae », Advances in Food and Nutrition Research, (2011) Chapter 9, 64 pp 111-128]. Chez l'algue brune, la fucoxanthine a une fonction biologique importante de protection contre le stress oxydatif au niveau de l'antenne collectrice permettant la photosynthèse [D.Siefermann-Harms, "The light-harvesting and protective functions of carotenoids in photosynthetic membranes" in: Physiologia Plantarum, (1987) Vol. 69(3) pp 561-568]. La synthèse de cette molécule dans l'algue est régulée finement en fonction des conditions de lumière [R. Goss, T. Jakob, "Regulation and fonction of xanthophyll cycle-dependent photoprotection in algae" in: Photosynthesis Research. (2010), Vol. 106(1-2) pp 103-122]. La fucoxanthine est au même titre que le gluthation réduit, l'$\alpha$-tocopherol, le $\beta$-carotene et les flavonoïdes, une réponse aux stress oxydatifs globaux [N. Mallick, F. Mohn, « Reactive oxygen species: response of algal cells » in : J. Plant Physiology. (2000) Vol. 157(2), pp 183-193]. La fucoxanthine a été largement décrite comme possédant les effets biologiques suivants : anti-oxydant, anti-obésité, anti-cancer, anti-diabétique, anti-photo-vieillissement, protection du système cardiovasculaire, anti-inflammatoire, neuroprotection, anti-angiogénique, anti-tyrosinase (sous-entendant un effet dépigmentant), ou un effet de prévention de l'ostéoporose [Kim et Pangestuti, « Biological activities and potential health benefits of fucoxanthin derived from marine brown algae », Advances in Food and Nutrition Research, (2011) Chapter 9, 64 pp 111-128 ; D'Orazio et al., "Fucoxanthin: a treasure from the sea" in : Marine Drugs, (2012), 10 pp 604-616.]. De par ces bénéfices pour la santé et la peau, la fucoxanthine, ou des extraits contenant de la fucoxanthine, sont largement utilisés dans divers domaines tels que la nutrition, la cosmétique ou la pharmacie.

La demande de brevet français publiée sous le numéro 2 837 383 divulgue que les extraits d'*Undaria pinnatifida,* présentaient des effets *in-vitro* positifs vis-à-vis des agressions occasionnées à la peau (espèces réactives oxygénées, métaux lourds, dioxyde de carbone, fumée de tabac, pollution chimique, ...) ; lesdits extraits d'algues étant des extraits aqueux obtenus à partir de thalles ou toute partie de thalles sous forme fraîche, congelée, sèche, entière, fragmentée ou broyée. Cependant, de tels extraits aqueux conduisent à un effet qui n'est pas suffisamment satisfaisant pour une application performante en cosmétique ou en pharmacie.

La demande de brevet français publiée sous le numéro 2 880 803 divulgue l'utilisation d'algues brune pour l'obtention d'un effet anti-âge. Cependant l'extrait mis en oeuvre dans cette demande de brevet conduit à un effet qui n'est pas suffisamment satisfaisant pour une application potentielle en cosmétique ou en pharmacie. De plus cet extrait est difficilement incorporable dans les formulations cosmétiques telles que les crèmes.

C'est pourquoi les inventeurs se sont attachés à développer un nouvel extrait d'algue brune ayant un effet anti-âge amélioré et facilement incorporable dans les formulations cosmétiques se présentant sous la forme de crèmes.

Selon un premier aspect, l'invention a pour objet un procédé d'obtention d'un extrait lipophile de gamétophytes

d'algue brune obtenu par le procédé comprenant les étapes successives suivantes :

- Une étape A) de préparation d'une suspension hydro-alcoolique de cellules de gamétophytes par mélange d'une suspension aqueuse de cellules de gamétophytes d'algue brune, avec au moins un alcool aliphatique comportant de un à quatre atomes de carbone ;
- Une étape B) de mélange de ladite suspension hydro-alcoolique de cellules de gamétophytes d'algue obtenue à l'étape A), avec au moins un triglycéride d'acides gras qui comportent de huit à vingt-deux atomes de carbone ;
- Une étape C) d'addition d'eau au mélange multiphasique obtenu à l'étape B) ;
- Une étape D) d'isolement dudit extrait lipophile de gamétophytes d'algue brune, du mélange obtenu à l'étape C).

Dans la définition de l'extrait lipophile de gamétophytes d'algue brune, objet de la présente invention, la suspension aqueuse de cellules de gamétophytes d'algue brune mise en oeuvre à l'étape A du procédé pour son obtention peut être préparée comme suit :
- Selon une étape a), on récolte des lames de sporophytes matures prêts à sporuler de l'algue brune utilisée. Les sporophytes matures sont des sporophytes comportant des zones fertiles.
- Selon une étape b), les sporophytes matures récoltés lors de l'étape a), sont disposés dans des bacs contenant de l'eau de mer et libèrent leurs spores dans le milieu. Les spores ainsi libérées entament leur germination pour donner naissance à des cellules de gamétophytes.
- Selon une étape c), les gamétophytes formés lors de l'étape b) sont isolés puis déposés dans un récipient contenant de l'eau de mer contenant au moins une source d'azote telle que le nitrate de sodium ($NaNO_3$) à une concentration comprise entre 50 et 250 mg/l avec une préférence pour 150mg/l et une source de phosphore telle que le dihydrogénophosphate de sodium ($NaH_2PO_4$) à une concentration comprise entre 5 et 75 mg/l avec une préférence pour 50 mg/l. selon un mode particulier de cette étape c), la suspension aqueuse ainsi formée est aussi additionnée d'autres éléments minéraux par ajout d'un milieu nutritif tel que le milieu de Provasoli de composition suivante :

| Milieu de Provasoli | |
|---|---|
| NaNO3 | 350mg |
| glycérophosphate de sodium | 50mg |
| $Fe(NH_4)_2(SO_4)_2$, $6H_2O$ | 18mg |
| $Na_2$ EDTA | 15mg |
| $H_3BO_3$ | 28,5mg |
| $FeCl_3$, $6H_2O$ | 1,225mg |
| $MnSO_4$, $H_2O$ | 4,1mg |
| $ZnSO_4$, $7H_2O$ | 0,55mg |
| $CoSO_4$, 7H2O | 0,12mg |
| Vitamine B12 | 10μg |
| Thiamine | 0,5mg |
| Biotine | 5μg |
| Tris buffer | 500mg |
| eau distillée | 100ml |

- Selon une étape d), Les gamétophytes sont cultivés dans des cuves de culture translucides sous un bullage d'air optionnellement additionné de dioxyde de carbone, à température ambiante sous illumination constante. Après 14 jours de culture, les cellules se sont multipliées et la quantité de biomasse est élevée. Il convient alors de récolter les cellules de gamétophytes présentes en culture en filtrant le contenu de la cuve sur un tamis filtrant au seuil de coupure 80μm gardant à sa surface les gamétophytes. Les gamétophytes d'algue brune obtenus sont ensuite rincés à l'eau de mer.
- Selon une étape e), les gamétophytes recueillis ainsi rincés, sont mis en suspension aqueuse. Cette suspension aqueuse est alors mise en oeuvre à l'étape A) du procédé d'obtention dudit extrait lipophile de gamétophytes d'algue brune.

Lors de l'étape A) du procédé d'obtention dudit extrait lipophile de gamétophytes d'algue brune tel que défini précédemment, la suspension aqueuse de cellules de gamétophytes est mélangée à l'alcool ou au mélange d'alcools

à une température de 20°C pendant au moins une heure, à raison de 5 à 30 litres d'alcool par kilogramme de biomasse et plus particulièrement à raison d'environ 10 litres d'alcool par kilogramme de biomasse. Ledit au moins un alcool aliphatique comportant de un à quatre atomes de carbone mis en oeuvre à l'étape A) du procédé d'obtention dudit ledit extrait lipophile de gamétophytes d'algue brune tel que défini précédemment est plus particulièrement choisi parmi l'éthanol, le propanol, l'isopropanol, le butanol, l'isobutanol ou un mélange de ces alcools ; il s'agit tout particulièrement de l'éthanol.

Lors de l'étape B) du procédé d'obtention dudit extrait lipophile de gamétophytes d'algue brune tel que défini précédemment, ladite suspension hydro-alcoolique de cellules de gamétophytes d'algue brune obtenue à l'étape A), est mélangée avec au moins un triglycéride d'acides gras qui comportent de huit à vingt-deux atomes de carbone. Ledit au moins un triglycéride d'acide gras comportant de huit à vingt-deux atomes de carbone, est plus particulièrement un mélange de triglycérides d'acides gras qui comportent de huit à dix atomes de carbone. Selon cette étape B), le mélange est agité pendant au moins une heure.

Selon un autre aspect particulier, ladite suspension hydro-alcoolique de cellules de gamétophytes d'algue brune obtenue à l'étape A), est mélangée avec ledit au moins un triglycéride d'acides gras qui comportent de huit à vingt-deux atomes de carbone de manière à ce que le ratio massique : masse de cellules de gamétophytes d'algue brune sur masse de triglycérides d'acides gras qui comportent de huit à vingt-deux atomes de carbone soit supérieur ou égal à 2% et inférieur ou égal à 10% et, de façon plus particulière, supérieur ou égal à 5%. Selon un aspect tout particulier, ce ratio massique est environ égal à 6%.

Lors de l'étape C) du procédé d'obtention dudit ledit extrait lipophile de gamétophytes d'algue brune tel que défini précédemment, le mélange multiphasique obtenu à l'étape B) est additionnée d'eau à une température de 20°C sous agitation.

Selon un autre aspect particulier, ledit mélange multiphasique obtenu à l'étape B) est additionnée d'eau de manière à ce que le ratio massique : masse de cellules de gamétophytes d'algue brune sur masse d'eau soit supérieur ou égal à 0,5% et inférieur ou égal à 2%. Selon un aspect tout particulier, ce ratio massique est environ égal à 1%.

L'étape D) d'isolement dudit extrait lipophile de gamétophytes d'algue brune, du mélange obtenu à l'étape C), est généralement effectuée de la manière suivante : le mélange ainsi formé est filtré pour éliminer la biomasse. Le filtrat est ensuite séparé par centrifugation pour obtenir l'extrait lipophile d'intérêt.

Par «algue brune », on désigne dans l'extrait lipophile objet de la présente invention, les éléments du groupe constitué par les algues brunes des ordres des *Ascoseirales, Asterocladales, Desmarestiales, Dictyotales, Dictyotophycidae, Discosporangiales, Discosporangiophycidae, Ectocarpales, Fucales, Fucophycidae, Ishigeales, Ishigeophycidae, Laminariales, Nemodermatales, Onslowiales, Phaeophyceae ordo incertae sedis, Phaeosiphoniellales, Ralfsiales, Scytothamnales, Sphacelariales, Sporochnales, Syringodermatales, Tilopteridales,* et plus particulièrement les éléments du groupe constitué par les algues brunes des ordres *Laminariales, Fucales, Desmarestiales*, *Ectocarpales* et *Tilopteridales,* et encore plus particulièrement les algues brunes de l'ordre *Laminariales.*

Dans l'extrait lipophile objet de la présente invention, on désigne plus particulièrement par « algue brune », les algues brunes de l'ordre *Laminariales* choisie parmi les algues brunes de la famille *Alariaceae* et de la famille *Laminariaceae.*

Dans l'extrait lipophile objet de la présente invention, on désigne plus particulièrement par « algue brune », les algues brunes de l'ordre *Laminariales* de la famille *Laminariaceae,* choisies parmi le groupe constitué par la *Laminaria digitata,* la *Laminaria saccharina,* la *Laminaria hyperborea* et la *Laminaria ochroleuca.*

Dans l'extrait lipophile objet de la présente invention, on désigne plus particulièrement par « algue brune », l'algue brune de l'ordre *Laminariales* de la famille *Alariaceae* qui est l'algue brune *Undaria Pinnatifida.*

De façon, optionnelle, après le rinçage, les gamétophytes obtenus à l'étape d) de préparation de la suspension aqueuse de cellules de gamétophytes d'algue brune mise en oeuvre à l'étape A) du procédé d'obtention dudit extrait lipophile de gamétophytes d'algue brune tel que défini précédemment, sont lyophilisés, généralement dans un lyophilisateur à plaques, puis broyés pour obtenir une poudre de lyophilisat de gamétophytes d'algue brune. C'est pourquoi, selon un autre aspect particulier, le procédé d'obtention dudit extrait lipophile de gamétophytes d'algue brune tel que défini précédemment, comprend en outre :

- Une étape $A_0$) de préparation de ladite suspension aqueuse de cellules de gamétophytes d'algue brune mise en oeuvre à l'étape A), par réhydratation d'une poudre de lyophilisat de cellules de gamétophytes d'algue brune.

L'étape $A_0$ telle que définie ci-dessus est généralement effectuée aussitôt après le broyage les cellules lyophilisées en mélangeant la poudre à l'eau de manière à obtenir une biomasse contenant notamment de 5% à 50% massique d'extrait sec, plus particulièrement de 15% à 45% massique d'extrait sec et tout particulièrement environ 35% d'extrait sec.

L'extrait lipophile de cellules d'algues brunes obtenu à l'issue de l'étape D) du procédé d'obtention dudit extrait lipophile de gamétophytes d'algue brune tel que défini précédemment, peut encore contenir de l'eau ; il est alors nécessaire d'assécher cette solution en ajoutant un sel de séchage, par exemple du sulfate de sodium anhydre ($NaSO_4$).

L'extrait lipophile de cellules d'algues brunes sec est ensuite filtré sur filtre papier en matière cellulosique. C'est pourquoi, selon un autre aspect particulier, le procédé d'obtention dudit extrait lipophile de gamétophytes d'algue brune tel que défini précédemment, comprend en outre :

- Une étape E) de séchage dudit extrait lipophile de gamétophytes d'algue brune, obtenu à l'étape D).

Selon un autre aspect plus particulier, l'extrait tel que défini précédemment est caractérisé en ce que les cellules de gamétophytes d'algue brune mises en oeuvre dans le procédé d'obtention, sont issues d'algues brunes de l'ordre *Laminariales* choisies parmi les algues brunes de la famille *Alariaceae* et de la famille *Laminariaceae,* plus particulièrement issues des algues brunes issues du groupe constitué par la *Laminaria digitata,* la *Laminaria saccharina,* la *Laminaria hyperborea,* la *Laminaria ochroleuca* et l' *Undaria Pinnatifida.*

Selon un autre aspect encore plus particulier, l'extrait tel que défini précédemment est caractérisé en ce que les cellules de gamétophytes d'algue brune mises en oeuvre dans le procédé d'obtention, sont issues de l'algue *Undaria pinnatifida.*

L'invention a aussi pour objet le procédé d'obtention de l'extrait lipophile de gamétophytes d'algue brune, tel que défini précédemment comprenant les étapes successives suivantes :

- Une étape A) de préparation d'une suspension hydro-alcoolique de cellules de gamétophytes par mélange d'une suspension aqueuse de cellules de gamétophytes d'algue brune, avec au moins un alcool aliphatique comportant de un à quatre atomes de carbone ;
- Une étape B) de mélange de ladite suspension hydro-alcoolique de cellules de gamétophytes d'algue obtenue à l'étape A), avec au moins un triglycéride d'acides gras qui comportent de huit à vingt-deux atomes de carbone ;
- Une étape C) d'addition d'eau au mélange multiphasique obtenu à l'étape B) ;
- Une étape D) d'isolement dudit extrait lipophile de gamétophytes d'algue brune, du mélange obtenu à l'étape C) ;

Selon des modes plus particuliers du procédé tel que défini ci dessus, il comprend en outre l'une ou l'autre ou l'ensemble des étapes suivantes :

- Une étape E) de séchage dudit extrait lipophile de gamétophytes d'algue brune, obtenu à l'étape D) ;
- Une étape $A_0$) de préparation de ladite suspension aqueuse de cellules de gamétophytes d'algue brune mise en oeuvre à l'étape A), par réhydratation d'un lyophilisat de cellules de gamétophytes d'algue brune.

Selon d'autres modes particuliers du procédé tel que défini ci-dessus :

- Lors de l'étape A), ledit au moins un alcool aliphatique comportant de un à quatre atomes de carbone est choisi parmi l'éthanol, le propanol, l'isopropanol, le butanol, l'isobutanol ou un mélange de ces alcools ; et il s'agit plus particulièrement de l'éthanol ; et/ou :

  - Lors de l'étape B), ledit au moins un triglycéride d'acide gras comportant de huit à vingt-deux atomes de carbone, est un mélange de triglycérides d'acides gras qui comportent de huit à dix atomes de carbone ; et/ou :
  - Lors de l'étape B), le ratio massique : masse de cellules de gamétophytes d'algue brune sur masse de triglycérides d'acides gras qui comportent de huit à vingt-deux atomes de carbone est supérieur ou égal à 2% et inférieur ou égal à 10% ; et il est plus particulièrement supérieur ou égal à 5% ; et/ou :

- Les cellules de gamétophytes d'algue brune mises en oeuvre sont issues des algues brunes de l'ordre *Laminariales* choisies parmi les algues brunes de la famille *Alariaceae* et de la famille *Laminariaceae,* plus particulièrement issues des algues brunes issues du groupe constitué par la *Laminaria digitata,* la *Laminaria saccharina,* la *Laminaria hyperborea,* la *Laminaria ochroleuca* et l' *Undaria Pinnatifida,* et plus particulièrement issue de l'algue *Undaria Pinnatifida.*

L'invention décrit aussi l'utilisation de l'extrait lipophile de gamétophytes d'algue brune tel que défini précédemment, dans le but d'empêcher ou de ralentir l'apparition des signes du vieillissement de la peau humaine ou des lèvres ou bien d'éliminer lesdits signes, ladite utilisation étant dans une composition cosmétique, ainsi qu'un procédé dans le but d'empêcher ou de ralentir l'apparition des signes du vieillissement de la peau humaine ou des lèvres ou bien d'éliminer lesdits signes, comprenant au moins une étape d'application sur la peau humaine ou sur les lèvres, d'une composition cosmétique (C1) à usage topique comprenant au moins un excipient cosmétiquement acceptable et une quantité efficace de l'extrait lipophile de gamétophytes d'algue brune tel que défini précédemment.

L'invention a aussi pour objet la composition (C1) telle que définie ci-dessus.

Dans le procédé tel que défini ci-dessus, ladite composition (C1) est généralement étalée sur la surface de la peau à traiter, puis la peau est massée quelques instants.

L'expression "à usage topique" utilisée dans la définition de la composition (C1) objet de la présente invention, signifie que ladite composition (C1) est mise en oeuvre par application sur la peau, qu'il s'agisse d'une application directe ou d'une application indirecte lorsque ladite composition (C1) selon l'invention est imprégnée sur un support destiné à être mis en contact avec la peau (papier, lingette, textile, dispositif transdermique, etc...).

L'expression « cosmétiquement acceptable » utilisée dans la définition de la composition (C1) objet de la présente invention, signifie selon la directive du Conseil de la Communauté Economique Européenne N°76/768/CEE du 27 juillet 1976 modifiée par la directive N°93/35/CEE du 14 juin 1993, qu'elle comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Par quantité efficace de l'extrait lipophile de gamétophytes d'algue brune tel que défini précédemment, on entend pour 100% de la masse de ladite composition (C1), la quantité comprise entre 0,1% et 5% massique, plus particulièrement entre 0,1% et 3% massique, et encore plus particulièrement entre 0,5% et 2 % massique dudit extrait lipophile de gamétophytes d'algue brune.

La composition (C1) objet de la présente invention, se présente généralement sous forme d'une solution aqueuse ou hydro-alcoolique ou hydro-glycolique, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

La composition (C1) objet de la présente invention, peut être conditionnée dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, l'extrait lipophile de gamétophytes d'algue brune objet de la présente invention, est associé à des additifs chimiques habituellement mis en oeuvre dans le domaine des formulations à usage topique, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales les agents plastifiants, les agents émulsionnants et co-émulsionnants, les agents opacificants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents que l'on peut associer à l'extrait lipophile de gamétophytes d'algue brune dans la composition (C1), on peut citer les tensioactifs moussants et/ou détergents anioniques, cationiques, amphotères ou non ioniques.

Parmi les tensioactifs anioniques moussants et/ou détergents, on peut citer les sels de métaux alcalins, de métaux alcalino-terreux, d'ammonium, d'amines, ou d'aminoalcools d'alkylether sulfates, d'alkyl sulfates, d'alkylamidoéther sulfates, d'alkylaryl polyéthersulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents, on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents, on peut citer particulièrement les dérivés d'ammoniums quaternaires.

Parmi les tensioactifs non ioniques moussants et/ou détergents, on peut citer plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer à l'extrait lipophile de

gamétophytes d'algue brune dans la composition (C1), on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants que l'on peut associer à l'extrait lipophile de gamétophytes d'algue brune dans la composition (C1), on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (VIII) :

$$CH_2=C(R'_3)-C(=O)-[CH_2-CH_2-O]_n-R'_4 \qquad (VIII)$$

dans laquelle $R'_3$ représente un atome d'hydrogène ou un radical méthyle, $R'_4$ représente un radical alkyle linéaire ou ramifié comportant de huit à trente atomes de carbone et n représente un nombre supérieur ou égal à un et inférieur ou égal à cinquante.

Les polymères de type polyélectrolytes, linéaires ou branchés ou réticulés que l'on peut associer à l'extrait lipophile de gamétophytes d'algue brune dans la composition (C1), peuvent se présenter sous la forme d'une solution, d'une suspension aqueuse, d'une émulsion eau-dans-huile, d'une émulsion huile-dans-eau, d'une poudre. Les polymères de type polyélectrolytes, linéaires ou branchés ou réticulés que l'on peut associer à l'extrait lipophile de gamétophytes d'algue brune dans la composition (C1), peuvent être sélectionnés parmi les produits commercialisés sous les appellations SIMULGEL™ EG, SIMULGEL™EPG, SEPIGEL™ 305, SIMULGEL™ 600, SIMULGEL™ NS, SIMULGEL™ INS 100, SIMULGEL™ FL, SIMULGEL™ A, SIMULGEL™ SMS 88, SEPINOV™EMT 10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC 2, ARISTOFLEX™HMB, COSMEDIA™SP, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100.

Comme exemples d'agents épaississants et/ou gélifiants que l'on peut associer à l'extrait lipophile de gamétophytes d'algue brune dans la composition (C1), on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1).

Comme exemples d'agents épaississants et/ou gélifiants que l'on peut associer à l'extrait lipophile de gamétophytes d'algue brune dans la composition (C1), on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer à l'extrait lipophile de gamétophytes d'algue brune dans la composition (C1), on peut citer la cellulose, les dérivés de cellulose comme la méthylcellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à l'extrait lipophile de gamétophytes d'algue brune dans la composition (C1), on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer à l'extrait lipophile de gamétophytes d'algue brune dans la composition (C1), on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer à l'extrait lipophile de gamétophytes d'algue brune dans la composition (C1), on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à l'extrait lipophile de gamétophytes d'algue brune dans la composition (C1), on peut citer les xylènes sulfonates, les cumènes sulfonates, l'hexyl polyglucoside, le (2-éthyl hexyl) polyglucoside ou le n-heptyl polyglucoside.

Comme exemples d'agents tensioactifs émulsionnants que l'on peut associer à l'extrait lipophile de gamétophytes d'algue brune dans la composition (C1), on peut citer les tensioactifs non ioniques, des tensioactifs anioniques, des tensioactifs cationiques.

Comme exemples de tensioactifs non-ioniques émulsionnants que l'on peut associer à l'extrait lipophile de gamétophytes d'algue brune dans la composition (C1), on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre cinq moles et cent cinquante moles d'oxyde d'éthylène, comme la composition comprenant de l'acide stéarique éthoxylé à cent trente-cinq moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters de méthyl glucoside ; les alkyl polyglycosides comportant un radical aliphatique, linéaire ou ramifié, saturé ou insaturé, et comportant de quatorze à trente-six atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le (2-octyl dodécyl) polyxyloside, le (12-hydroxy stéaryl) polyglucoside ; les compositions d'alcools gras linéaires ou ramifiés, saturés ou insaturés, et comportant de quatorze à trente-six atomes de carbone, et d'alkyl polyglycosides tels que décrits précédemment, par exemple les compositions commercialisées sous les noms de marque MONTANOV™68, MONTANOV™14, MONTANOV™82, MONTANOV™202, MONTANOV™S, MONTANOV™WO18, MONTANOV™L, FLUIDANOV™20X et EASYNOV™.

Comme exemples de tensioactifs anioniques que l'on peut associer à l'extrait lipophile de gamétophytes d'algue brune dans la composition (C1), on peut citer le glycéryl stéarate citrate, le cétéarylsulfate, les savons comme le stéarate de sodium ou le stéarate de triéthanolammonium, les dérivés N-acylés d'acides aminés salifiés comme par exemple le stéaroyl glutamate.

Comme exemples de tensioactifs cationiques émulsionnants que l'on peut associer à l'extrait lipophile de gamétophytes d'algue brune dans la composition (C1), on peut citer les aminoxydes, le quaternium-82 et les tensioactifs décrits dans la demande de brevet WO 96/00719 et principalement ceux dont la chaîne grasse comprend au moins seize atomes de carbone.

Comme exemples d'agents opacifiants et/ou nacrants que l'on peut associer à l'extrait lipophile de gamétophytes d'algue brune dans la composition (C1), on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de douze à vingt-deux atomes de carbone.

Comme exemples d'agents de texture que l'on peut associer à l'extrait lipophile de gamétophytes d'algue brune dans la composition (C1), on peut citer des dérivés N-acylés d'acides aminés, comme la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica.

Comme exemples d'agents déodorants que l'on peut associer à l'extrait lipophile de gamétophytes d'algue brune dans la composition (C1), on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles que l'on peut associer à l'extrait lipophile de gamétophytes d'algue brune dans la composition (C1), on peut citer les huiles minérales telles que l'huile de paraffine, l'huile de vaseline, les isoparaffines ou les huiles blanches minérales ; les huiles d'origine animale, telles que le squalène ou le squalane ;les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer à l'extrait lipophile de gamétophytes d'algue brune dans la composition (C1), on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer à l'extrait lipophile de gamétophytes d'algue brune dans la composition (C1), on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecylènoyl phénylalanine commercialisé sous l'appellation SEPIWHITE™MSH, le SEPICALM™VG, le mono ester et/ou le diester de glycérol du ω-undecelynoyl phénylalanine, les ω-undecylènoyl dipeptides, l'arbutine, l'acide kojique, l'hydroquinone ; les composés montrant une action apaisante notamment le SEPICALM™ S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylglucoside, la composition commercialisée sous le nom de marque AQUAXYL™, la composition commercialisés sous le nom de marque PRO-XYLANE™, les dérivés de C-glycosides et plus particulièrement les dérivés de C-glucosides, de C-xylosides ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés comme le MATRIXIL™ ; les acides aminés N-acylés ; les hydrolysâts partiels

de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5, le FLUIDIPURE™8G ; l'OCTOPIROX™ ou le SENSIVA™ SC50 ; les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule ; les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caraténoïdes (et plus particulièrement le beta carotène et le gamma carotène), le produit commercialisé sous le nom de marque « Carrot oil » (Nom INCI : Daucus Carota, helianthus annuus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI : butylene glycol and Acetyl Tyrosine and hydrolyzed vegetable protein and Adenosine triphosphate) par la société UNIPEX, le produit commercialisé sous le nom de marque « Try-Excell™ » (nom INCI : Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™» (nom INCI : hydrolyzed wheat protein and acetyl tyrosine and copper gluconate) par la société Alban Muller, le produit commercialisé sous le nom de marque Tyrostan™ (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze™ (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI : Butylene glycol , Palmitoyl Tripeptide-40) par la société LIPOTEC, les sucres et les dérivés de sucres par exemple le produit commercialisé sous le nom de marque Tanositol™ (nom INCI : inositol) par la société Provital, le produit commercialisé sous le nom de marque Thalitan™ (ou Phycosaccharide™ AG) par la société CODIF international (nom INCI : Aqua and hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI : Maltodextrin, Mucuna Pruriens Seed extract) par la société Alban Muller, les composés riches en flavonoïdes par exemple le produit commercialisé sous le nom de marque « Biotanning » (nom INCI : Hydrolyzed citrus Aurantium dulcis fruit extract) par la société Silab et connu pour être riche en flavonoides de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis

parmi ceux décrits dans la demande de brevet européen publiée sous le numéro EP 1 515 688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, , des associations de mono- et diester d'acide cinnamique et de vitamine C, et plus généralement ceux cités dans la demande de brevet européen publiée sous le numéro EP 1 515 688 A2.

Comme exemples d'agents antioxydants que l'on peut associer à l'extrait lipophile de gamétophytes d'algue brune dans la composition (C1), on peut citer l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE™ GL 47S commercialisé par la société Akzo Nobel sous le nom INCI : Tetrasodium Glutamate Diacetate. Comme exemples de filtres solaires que l'on peut associer à l'extrait lipophile de gamétophytes d'algue brune dans la composition (C1), on peut citer tous ceux figurant dans la directive cosmétique 76/768/CEE modifiée annexe VII.

Parmi les filtres organiques solaires que l'on peut associer à l'extrait lipophile de gamétophytes d'algue brune dans la composition (C1), on peut citer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de (p-isopropanol phényle) ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle, le cinnamate de (p-méthoxy 2-éthylhexyle), le cinnamate de (p-méthoxy 2-éthoxy éthyle), le cinnamate de (p-méthoxy cyclohexyle), le cinnamate d'éthyl-$\alpha$-cyano-$\beta$-phényle, le cinnamate de (2-éthyl hexyl)-$\alpha$-cyano-$\beta$-phényle, le cinnamate de diparaméthoxy mono-2-éthylhexanoyl de glycéryle ; la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le (2-éthyl hexyl) 4'-phényl benzophénone-2-carboxylate, la 2-hydroxy 4-(n-octyloxy) benzophénone, la 4-hydroxy 3-carboxy benzophénone ; le 3-(4'-méthyl benzylidène) d,l-camphre, le 3-(benzylidène) d,l-camphre, le benzalkonium méthosulfate camphre ; l'acide urocanique, l'urocanate d'éthyle ; la famille des dérivés de l'acide sulfonique comme l'acide sulfonique 2-phényl benzimidazole-5 et ses sels ; la famille des dérivés de la triazine comme l'hydroxyphényl triazine, l'(éthylhexyloxy) (hydroxyphényl) (4-méthoxy phényl) triazine, le 2,4,6-trianillino-(p-carbo-2'-éthylhexyl-1'-oxy)-1,3,5-triazine, le 4,4-((6-(((1,1-diméthyl éthyl) amino) carbonyl) phenyl) amino)-1,3,5-triazine-2,4-diyl diimino) bis(2-éthyl hexyl) ester de l'acide benzoïque, le 2-phényl-5-méthyl benzoxazole, le 2-(2'-hydroxy 5'-méthyl phényl) benzotriazole, le 2-(2'-hydroxy 5'-t-octyl phényl) benzotriazole, le 2-(2'-hydroxy 5'-méthy phényl) benzotriazole; la dibenzazine; le dianisoylméthane, le 4-méthoxy 4"-t-butyl benzoyl méthane ; la 5-(3,3-diméthyl 2-norbornylidène)-3-pentan-2-one ; la famille des dérivés du diphénylacrylate comme le (2-éthyl hexyl) 2-cyano 3,3-diphényl 2-propènoate, l'éthyl-2-cyano 3,3-diphényl 2-propènoate ; la famille des polysiloxanes comme le malonate de benzylidène siloxane.

Parmi les filtres inorganiques solaires, également appelés "écrans minéraux", que l'on peut associer à l'extrait lipophile de gamétophytes d'algue brune dans la composition (C1), on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

L'invention a aussi pour objet l'extrait lipophile de gamétophytes d'algue brune, tel que défini précédemment, pour son utilisation dans une méthode de traitement thérapeutique des signes de vieillissement de la peau humaine ou des lèvres, appliquée au corps humain.

Les exemples suivants illustrent l'invention sans toutefois la limiter.

**Listes des extraits testés**

Extrait A (selon l'invention) : Extrait de gamétophytes d'*Undaria pinnatifida*

Extrait B (selon l'état de la technique) : Extrait BB de l'exemple 3 de la demande de brevet français FR 2 880 803 A1

Extrait C (selon l'état de la technique : extrait de sporophyte d'*Undaria pinnatifida,*
obtenu par extraction dans un mélange d'eau et de glycérol.

**Efficacité biologique des extraits testés**

Etude *in-vitro*

Le modèle choisi pour mettre en évidence l'effet technique de l'extrait selon l'invention est un modèle d'étude de l'expression de gènes sur explants de peau humaine. Les tests dits génomiques ou transcriptomiques sont très largement utilisés dans différents domaines tels que la cosmétique afin de mettre en évidence des bénéfices biologiques. Le travail sur explants de peau humaine permet d'être dans des conditions plus physiologiques que le travail sur cultures de cellules en monocouche.

Des explants de peau humaine d'environ 10mm de diamètre, issus de 2 donneurs caucasiens et de sexe féminin (49 ans et 46 ans), provenant de déchets opératoires abdominaux) ont été prélevés. Ils ont été ensuite mis en survie dans un milieu spécifique BEM (Bio-Ec's Explants Medium) à 37°C en atmosphère humide à 5% de $CO_2$. Les produits indiqués dans le tableau 1 ont ensuite été appliqués pour une durée de 24h.

**Tableau 1** : Modalités d'application des produits.

| Extrait | Aspect physique | Concentration appliquée en topique | Concentration appliquée dans le milieu de culture |
|---|---|---|---|
| A | Liquide vert foncé | 1% | 0,1% |
| B | Liquide Vert clair | 1% | 0,1% |
| C | Liquide incolore | 1% | 0,1% |
| Véhicule | Liquide incolore | 2,5% en gel aqueux | 1% |

Les produits ont été préparés dans un gel hydro-alcoolique à 2,5% d'éthanol pour les applications topiques, et avec 1% d'éthanol dans le milieu de culture afin d'être solubilisés. 3 explants par condition ont ainsi été préparés. Au bout de 24h les ARN totaux ont été extraits puis une analyse quantitative et qualitative a été menée afin de déterminer leur concentration et leur niveau de pureté et d'intégrité avant de leur faire subir une étape d'amplification. 50ng d'ARN ont donc été ensuite utilisés puis rétro-transcrits afin d'obtenir un ADN complémentaire. Celui-ci a ensuite été amplifié par la technique de PCR (Polymerase Chain Reaction) quantitative (qPCR) à l'aide de sondes spécifiques à 3 gènes: GAPDH (gène de ménage, contrôle), HIF-1a et SIRT-1, et d'un marqueur fluorescent, le SYBR Green permettant le suivi de la réaction d'amplification en temps réel. La PCR fonctionne par cycle et le nombre de cycles (Cq) effectué pour chaque cible, afin que celle-ci puisse être détectée, est reporté. Cette valeur de cycle est ensuite soustraite à celle de la GAPDH afin de normaliser les effets obtenus, puis un calcul de ratio d'expression par rapport à la condition véhicule est effectué (RQ).
Pour chaque extrait testé, et pour chaque gène d'intérêt, on calcule les grandeurs suivantes :

$$\Delta Cq \text{ (extrait i)} = Cq \text{ (gène d'intérêt)} - Cq \text{ (gène référence)},$$

avec :

- Cq (gène d'intérêt) représentant le nombre moyen de cycles effectués et nécessaires pour obtenir un signal pour un extrait donné et un gène d'intérêt donné ;
- Cq (gène référence) représentant le nombre moyen de cycles effectués et nécessaires pour obtenir un signal pour un extrait donné et un gène référence (ici, la GAPDH).

Pour chaque gène d'intérêt et pour chaque extrait testé, on calcule

$$\Delta\Delta Cq = \Delta Cq \text{ (extrait i)} - \Delta Cq \text{ du véhicule}.$$

Pour chaque extrait testé, et pour chaque gène d'intérêt, on calcule la valeur RQ selon :

$$RQ = 2^{-\Delta\Delta Cq}$$

Les résultats de variation sont présentés dans le tableau n°2 ci-dessous.

**Tableau 2** : Résultats d'expression de gène obtenus.

| | **GAPDH** | | **HIF-1a** | | **SIRT-1** | |
|---|---|---|---|---|---|---|
| **Extrait** | Nombre de cycles moyen | RQ moyen | Nombre de cycles moyen | RQ moyen | Nombre de cycles moyen | RQ moyen |
| A | 20,21 | 1 | 24,99 | **1,41** | 28,27 | **1,56** |
| B | 19,85 | 1 | 24,88 | 0,6 | 28,28 | 0,73 |
| C | 19,99 | 1 | 24,94 | 0,67 | 28,10 | 0,82 |
| Véhicule<br>(a)<br>(b) | <br>20,02<br>21,12 | | <br>25,38<br>25,41 | | <br>27,91<br>28,83 | |
| (a) : expérience effectuée avec le composé A<br>(b) : expérience effectuée avec les autres composés | | | | | | |

L'extrait A (extrait de gamétophytes d'*Undaria pinnatifida*) permet d'induire une surexpression des gènes HIF-1a et SIRT-1 relativement à la condition véhicule. Les autres composés ne permettent pas d'induire l'expression de ces gènes. Les présents résultats permettent de mettre en évidence l'effet technique supplémentaire de l'invention par rapport à l'état de la technique le plus proche représenté par l'extrait aqueux de sporophyte d'Undaria pinnatifida (extrait C), et par l'extrait BB de la demande de brevet FR 2 880 803 A1 (extrait B).

Etude *in-vivo*

Une étude clinique, réalisée sur un panel représentatif de sujets (25 femmes), a permis de démontrer un effet de réduction de la rugosité du micro-relief cutané et de la profondeur moyenne des rides au niveau de la zone de la « patte d'oie », associé à l'application sur la zone en question de la peau d'une formulation cosmétique comportant une quantité efficace d'extrait A selon l'invention comparativement à l'application, sur la même zone de la peau, d'une formulation cosmétique « *placebo* » ne comprenant pas ledit extrait A et ceci pendant deux mois.

Une autre étude clinique, réalisée sur un panel représentatif de sujets (panel mixte comprenant 20 personnes), a permis de démontrer un effet d'amélioration de la capacité anti-oxydante de la peau associé à l'application sur la peau d'une formulation cosmétique comportant une quantité efficace d'extrait A selon l'invention comparativement à l'application sur la peau, d'une formulation cosmétique « *placebo* » ne comprenant pas ledit extrait A, au terme de 14 et 28 jours d'applications. Cet effet a été démontré par la mise en oeuvre du test du pouvoir antioxydant réducteur du fer ferrique (dite méthode « FRAP »).

Une autre évaluation clinique a été effectuée sur ce même panel représentatif de sujets et a permis de démontrer un effet d'amélioration de la résistance de la peau face à un stress induit par une exposition aux rayonnements ultraviolets A, associé à l'application sur la peau d'une formulation cosmétique comportant une quantité efficace d'extrait A selon l'invention comparativement à l'application sur la peau, d'une formulation cosmétique «*placebo*» ne comprenant pas ledit extrait A, au terme de 14 et 28 jours d'applications. Cet effet a été démontré par la mise en oeuvre de la méthode de dosage de la Malondialdéhyde (ou « MDA »), principal produit de la peroxydation lipidique, par la méthode d'Erdelmeier et al., 1998, basée sur la capacité d'un chromogène, le NMPI (N-methyl-2-phenylindole) à réagir avec le MDA à 45°C et à pH acide pour produire un chromophore stable présentant un pic d'absorption à 586nm.

## Revendications

1. Procédé d'obtention d'un extrait lipophile de gamétophytes d'algue brune, comprenant les étapes successives suivantes :

   - Une étape A) de préparation d'une suspension hydro-alcoolique de cellules de gamétophytes par mélange d'une suspension aqueuse de cellules de gamétophytes d'algue brune, avec au moins un alcool aliphatique

comportant de un à quatre atomes de carbone ;
- Une étape B) de mélange de ladite suspension hydro-alcoolique de cellules de gamétophytes d'algue obtenue à l'étape A), avec au moins un triglycéride d'acides gras qui comportent de huit à vingt-deux atomes de carbone ;
- Une étape C) d'addition d'eau au mélange multiphasique obtenu à l'étape B) ;
- Une étape D) d'isolement dudit extrait lipophile de gamétophytes d'algue brune, du mélange obtenu à l'étape C).

**2.** Procédé tel que défini à la revendication 1, selon lequel, lors de l'étape A), ledit au moins un alcool aliphatique comportant de un à quatre atomes de carbone est choisi parmi l'éthanol, le propanol, l'isopropanol, le butanol, l'isobutanol ou un mélange de ces alcools.

**3.** Procédé tel que défini à la revendication 2, selon lequel, lors de l'étape A), ledit au moins un alcool aliphatique comportant de un à quatre atomes de carbone est l'éthanol.

**4.** Procédé tel que défini à l'une quelconque des revendications 1 à 3, selon lequel, lors de l'étape B), ledit au moins un triglycéride d'acide gras comportant de huit à vingt-deux atomes de carbone, est un mélange de triglycérides d'acides gras qui comportent de huit à dix atomes de carbone.

**5.** Procédé tel que défini à l'une quelconque des revendications 1 à 4, selon lequel, lors de l'étape B), le ratio massique : masse de cellules de gamétophytes d'algue brune sur masse de triglycérides d'acides gras qui comportent de huit à vingt-deux atomes de carbone est supérieur ou égal à 2% et inférieur ou égal à 10%.

**6.** Procédé tel que défini à la revendication 5, selon lequel, lors de l'étape B), ledit ratio massique : masse de cellules de gamétophytes d'algue brune sur masse de triglycérides d'acides gras qui comportent de huit à vingt-deux atomes de carbone est supérieur ou égal à 5%.

**7.** Procédé tel que défini à l'une quelconque des revendications 1 à 6, comprenant en outre une étape A0) de préparation de ladite suspension aqueuse de cellules de gamétophytes d'algue brune mise en oeuvre à l'étape A), par réhydratation d'un lyophilisat de cellules de gamétophytes d'algue brune.

**8.** Procédé tel que défini à la revendication 1, comprenant en outre :

- Une étape E) de séchage dudit extrait lipophile de gamétophytes d'algue brune, obtenu à l'étape D).

**9.** Procédé tel que défini à la revendication 7, comprenant en outre :

- Une étape E) de séchage dudit extrait lipophile de gamétophytes d'algue brune, obtenu à l'étape D).

**10.** Procédé tel que défini à l'une quelconque des revendications 1 à 9, dans lequel les cellules de gamétophytes d'algue brune mises en oeuvre sont issues de l'algue *Undaria pinnatifida.*

**11.** Procédé d'obtention d'un extrait lipophile de gamétophytes d'algue brune issues de l'algue *Undaria pinnatifida,* comprenant les étapes successives suivantes :

- Une étape A0) de préparation de ladite suspension aqueuse de cellules de gamétophytes d'algue brune mise en oeuvre à l'étape A), par réhydratation d'une poudre de lyophilisat de cellules de gamétophytes d'algue brune.
- Une étape A) de préparation d'une suspension hydro-alcoolique de cellules de gamétophytes par mélange d'une suspension aqueuse de cellules de gamétophytes d'algue brune, avec de l'éthanol ;
- Une étape B) de mélange de ladite suspension hydro-alcoolique de cellules de gamétophytes d'algue obtenue à l'étape A), avec au moins un mélange de triglycéride d'acides gras qui comportent de huit à dix-huit atomes de carbone, tel que le ratio massique : masse de cellules de gamétophytes d'algue brune sur masse de triglycérides d'acides gras qui comportent de huit à vingt-deux atomes de carbone est supérieur ou égal à 5% et inférieur ou égal à 10% ;
- Une étape C) d'addition d'eau au mélange multiphasique obtenu à l'étape B) ;
- Une étape D) d'isolement dudit extrait lipophile de gamétophytes d'algue brune, du mélange obtenu à l'étape C).
- Une étape E) de séchage dudit extrait lipophile de gamétophytes d'algue brune, obtenu à l'étape D).

**12.** Composition cosmétique comprenant une quantité efficace, l'extrait lipophile de gamétophytes d'algue brune, obtenu par le procédé tel que défini à l'une quelconque des revendications 1 à 11 et au moins un excipient cosmétiquement

acceptable.

## Patentansprüche

1. Verfahren zur Gewinnung eines lipophilen Extraktes von Braunalgen-Gametophyten, das nacheinander die folgenden Schritte umfasst:

   - einen Schritt A) des Herstellens einer wässrig-alkoholischen Suspension von Gametophytenzellen durch Mischen einer wässrigen Suspension von Braunalgen-Gametophytenzellen mit mindestens einem aliphatischen Alkohol, der ein bis vier Kohlenstoffatome einschließt;
   - einen Schritt B) des Mischens der in Schritt A) erhaltenen wässrig-alkoholischen Suspension von Algen-Gametophytenzellen mit mindestens einem Triglycerid von Fettsäuren, die acht bis zweiundzwanzig Kohlenstoffatome einschließen;
   - einen Schritt C) des Hinzufügens von Wasser zu dem in Schritt B) erhaltenen Mehrphasengemisch;
   - einen Schritt D) des Isolierens des lipophilen Extraktes von Braunalgen-Gametophyten aus dem in Schritt C) erhaltenen Gemisch.

2. Verfahren nach Anspruch 1, wobei in Schritt A) der mindestens eine aliphatische Alkohol, der ein bis vier Kohlenstoffatome einschließt, ausgewählt wird aus Ethanol, Propanol, Isopropanol, Butanol, Isobutanol oder eines Gemisches dieser Alkohole.

3. Verfahren nach Anspruch 2, wobei in Schritt A) der mindestens eine aliphatische Alkohol, der ein bis vier Kohlenstoffatome einschließt, Ethanol ist.

4. Verfahren nach einem der Ansprüche 1 bis 3, wobei in Schritt B) das mindestens eine Triglycerid von Fettsäuren, die acht bis zweiundzwanzig Kohlenstoffatome einschließen, ein Gemisch von Fettsäuretriglyceriden ist, die acht bis zehn Kohlenstoffatomen einschließen.

5. Verfahren nach einem der Ansprüche 1 bis 4, wobei in Schritt B) das Massenverhältnis: Masse von Braunalgen-Gametophytenzellen zu Masse von Fettsäuretriglyceriden, die acht bis zweiundzwanzig Kohlenstoffatome einschließen, größer oder gleich 2 % und kleiner oder gleich 10 % ist.

6. Verfahren nach Anspruch 5, wobei in Schritt B) das Massenverhältnis: Masse von Braunalgen-Gametophytenzellen zu Masse von Fettsäuretriglyceriden, die acht bis zweiundzwanzig Kohlenstoffatome einschließen, größer oder gleich 5 % ist.

7. Verfahren nach einem der Ansprüche 1 bis 6, das weiter einen Schritt A0) des Herstellens der in Schritt A) umgesetzten wässrigen Suspension von Braunalgen-Gametophytenzellen durch Rehydratisierung eines Lyophilisats von Braunalgen-Gametophytenzellen umfasst.

8. Verfahren nach Anspruch 1, das weiter umfasst:

   - einen Schritt E) des Trocknens des in Schritt D) erhaltenen lipophilen Extraktes von Braunalgen-Gametophyten.

9. Verfahren nach Anspruch 7, das weiter umfasst:

   - einen Schritt E) des Trocknens des in Schritt D) erhaltenen lipophilen Extraktes von Braunalgen-Gametophyten.

10. Verfahren nach einem der Ansprüche 1 bis 9, wobei die erhaltenen Gametophytenzellen von Braunalge von der Alge *Undaria pinnatifida* stammen.

11. Verfahren zur Gewinnung eines lipophilen Extraktes von Braunalgen-Gametophyten, welche von der Alge *Undaria pinnatifida* stammen, das nacheinander die folgenden Schritte umfasst:

    - einen Schritt A0) des Herstellens der in Schritt A) umgesetzten wässrigen Suspension von Braunalgen-Gametophytenzellen durch Rehydratisierung eines Lyophilisatpulvers von Braunalgen-Gametophytenzellen.
    - einen Schritt A) des Herstellens einer wässrig-alkoholischen Suspension von Gametophytenzellen durch

Mischen einer wässrigen Suspension von Braunalgen-Gametophytenzellen mit Ethanol;
- einen Schritt B) des Mischens der in Schritt A) erhaltenen wässrig-alkoholischen Suspension von Algen-Gametophytenzellen mit mindestens einem Gemisch von Triglycerid von Fettsäuren, die acht bis achtzehn Kohlenstoffatome einschließen, derart, dass das Massenverhältnis: Masse der Braunalgen-Gametophytenzellen zu Masse der Fettsäuretriglyceriden, die acht bis zweiundzwanzig Kohlenstoffatome einschließen, größer oder gleich 5 % und kleiner oder gleich 10 % ist;
- einen Schritt C) des Hinzufügens von Wasser zu dem in Schritt B) erhaltenen Mehrphasengemisch;
- einen Schritt D) des Isolierens des lipophilen Extraktes von Braunalgen-Gametophyten aus dem in Schritt C) erhaltenen Gemisch.
- einen Schritt E) des Trocknens des in Schritt D) erhaltenen lipophilen Extraktes von Braunalgen-Gametophyten.

**12.** Kosmetische Zusammensetzung, die eine wirksame Menge von lipophilem Extrakt aus Braunalgen-Gametophyten, erhalten durch das Verfahren nach einem der Ansprüche 1 bis 11, und mindestens einen kosmetisch akzeptablen Hilfsstoff umfasst.

## Claims

**1.** Method for obtaining a lipophilic extract of brown algae gametophytes, comprising the following successive steps:

- a step A) of preparing a water/alcohol suspension of gametophyte cells by mixing an aqueous suspension of brown algae gametophyte cells with at least one aliphatic alcohol containing one to four carbon atoms;
- a step B) of mixing the said water/alcohol suspension of algae gametophyte cells obtained in step A) with at least one triglyceride of fatty acids containing eight to twenty-two carbon atoms;
- a step C) of adding water to the multiphase mixture obtained in step B);
- a step D) of isolating the said lipophilic extract of brown algae gametophytes from the mixture obtained in step C).

**2.** Method according to claim 1, whereby, in step A), said at least one aliphatic alcohol containing one to four carbon atoms is selected from among ethanol, propanol, isopropanol, butanol, isobutanol or a mixture of these alcohols.

**3.** Method according to claim 2, whereby, in step A), said at least one aliphatic alcohol containing one to four carbon atoms is ethanol.

**4.** Method according to any one of claims 1 to 3, whereby, in step B), said at least one fatty acid triglyceride containing eight to twenty-two carbon atoms is a mixture of triglyceride of fatty acids containing eight to ten carbon atoms.

**5.** Method according to any one of claims 1 to 4whereby, in step B), the mass ratio: mass of brown algae gametophyte cells to the mass of triglycerides of fatty acids containing eight to twenty-two carbon atoms is greater than or equal to 2% and less than or equal to 10%.

**6.** Method according to claim 5, whereby, in step B), said mass ratio: mass of brown algae gametophyte cells to mass of triglycerides of fatty acids containing eight to twenty-two carbon atoms is greater than or equal to 5%.

**7.** Method according to any one of claims 1 to 6, further comprising a step A0) of preparing said aqueous suspension of brown algae gametophyte cells implemented in step A) by rehydrating a lyophilisate of brown algae gametophyte cells.

**8.** Method according to claim 1, further comprising:

- a step E) of drying said lipophilic extract of brown algae gametophytes obtained in step D).

**9.** Method according to claim 7, further comprising:

- a step E) of drying said lipophilic extract of brown algae gametophytes obtained in step D).

**10.** Method according to any one of claims 1 to 9, wherein the brown algae gametophyte cells used originate from the alga *Undaria pinnatifida.*

11. Method for obtaining a lipophilic extract of the brown algae gametophytes originating from the alga *Undaria pinnatifida,* comprising the following successive steps:

- a step A0) of preparing said aqueous suspension of brown algae gametophyte cells used in step A) by rehydrating a lyophilisate powder of brown algae gametophyte cells;
- a step A) of preparing a water/alcohol suspension of gametophyte cells by mixing an aqueous suspension of brown algae gametophyte cells with ethanol;
- a step B) of mixing said water/alcohol suspension of algae gametophyte cells obtained in step A) with at least one mixture of triglyceride of fatty acids containing eight to eighteen carbon atoms, such that the mass ratio: mass of brown algae gametophyte cells to the mass of triglycerides of fatty acids containing eight to twenty-two carbon atoms is greater than or equal to 5% and less than or equal to 10%;
- a step C) of adding water to the multiphase mixture obtained in step B);
- a step D) of isolating said lipophilic extract of brown algae gametophytes from the mixture obtained in step C).
- a step E) of drying said lipophilic extract of brown algae gametophytes obtained in step D).

12. Cosmetic composition comprising an effective amount of the lipophilic extract of brown algae gametophytes obtained by the method defined in any one of claims 1 to 11, and at least one cosmetically acceptable excipient.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2837383
- FR 2880803
- WO 9600719 A
- EP 0971683 A
- EP 1515688 A2
- FR 2880803 A1


### Littérature non-brevet citée dans la description


- **SHIN et al.** Chronic heat treatment causes skin wrinkle formation and oxidative damage in hairless mice. *Mech Ageing Dev,* 2012, vol. 133 (2-3), 92-8
- **CORSTJENS et al.** Prevention of oxidative damage that contributes to the loss of bioenergetic capacity in ageing skin. *Exp Gerontol,* 2007, vol. 42 (9), 924-9
- Biological activities and potential health benefits of fucoxanthin derived from marine brown algae. **KIM ; PANGESTUTI.** Advances in Food and Nutrition Research. 2011, vol. 64, 111-128
- **D.SIEFERMANN-HARMS.** The light-harvesting and protective functions of carotenoids in photosynthetic membranes. *Physiologia Plantarum,* 1987, vol. 69 (3), 561-568
- **R. GOSS ; T. JAKOB.** Regulation and fonction of xanthophyll cycle-dependent photoprotection in algae. *Photosynthesis Research,* 2010, vol. 106 (1-2), 103-122
- **N. MALLICK ; F. MOHN.** Reactive oxygen species: response of algal cells. *J. Plant Physiology,* 2000, vol. 157 (2), 183-193
- **KIM ; PANGESTUTI.** Biological activities and potential health benefits of fucoxanthin derived from marine brown algae. *Advances in Food and Nutrition Research,* 2011, (64), 111-128
- **D'ORAZIO et al.** Fucoxanthin: a treasure from the sea. *Marine Drugs,* 2012, vol. 10, 604-616